Europäisches Patentamt

**European Patent Office**

Office européen des brevets

(19)

(11) Publication number: **0 149 439**
**A2**

(12) # EUROPEAN PATENT APPLICATION

(21) Application number: **84870167.8**

(22) Date of filing: **04.12.84**

(51) Int. Cl.⁴: **C 07 C 67/313**
**C 07 C 69/716**

(30) Priority: **03.01.84 US 567591**

(43) Date of publication of application:
**24.07.85 Bulletin 85/30**

(84) Designated Contracting States:
**BE DE FR GB NL**

(71) Applicant: **Monsanto Company**
**Patent Department 800 North Lindbergh Boulevard**
**St. Louis Missouri 63167(US)**

(72) Inventor: **Rao, Anantaneni S. C. P.**
**1293 Mautenne Drive**
**Manchester Missouri 63011(US)**

(72) Inventor: **Dyroff, David Ray**
**15 Woodoaks Trail**
**Ladue Missouri 63124(US)**

(74) Representative: **Lunt, John Cooper et al,**
**Monsanto Europe S.A. Patent Department Avenue de**
**Tervuren 270-272 Letter Box No 1**
**B-1150 Brussels(BE)**

(54) Production of Alkyl glyoxylates.

(57) Alkyl glyoxylates are prepared by contacting gaseous alkyl glycolate in the presence of gaseous oxygen source with unsupported metallic silver at a temperature in the range of about 325° to about 685°C. The alkyl glyoxylates are useful as intermediates for the synthesis of other products such as allantoin.

EP 0 149 439 A2

## PRODUCTION OF ALKYL GLYOXYLATES
### BACKGROUND OF THE INVENTION

This invention relates to the catalytic dehydrogenation of alkyl glycolates in the presence of oxygen. More particularly, this invention relates to an improved process for the production of alkyl glyoxylate by contacting an alkyl glycolate in the gaseous phase with an oxygen source and a metallic silver catalyst. Alkyl glyoxylates are important intermediates for the synthesis of other products such as allantoin.

U.S.P. 1,614,195 issued to Alfred Haussler January 11, 1927 discloses a process for producing esters of oxyacids by the action of oxygen upon the corresponding hydroxyacid esters in the gaseous phase in the presence of catalysts containing metal oxides or salts of acids derived from metal oxides. The yields of oxyacid esters reported are in the range of 40 - 70 percent of the theoretical yield. The hydroxy acid esters employed are, for example, esters of lactic acid, glycolic acid, tartaric acid, etc.

U.S.P. 4,340,748 issued to Herbert Baltes et al July 20, 1982 discloses a process which produces alkyl glyoxylates at higher yields by dehydrogenation of glycolic acid esters in the gaseous phase in contact with a catalyst containing at least one of the elements V, Mo, Ag, or Cu, as well as at least one of the elements Sn, Sb, Bi or elements of the first main group or second main group of the Periodic Table as a promoter. While higher yields are desirable, the operating conditions described in 4,340,748 present such disadvantages as low weight hourly space velocity (WHSV) and a high degree of dilution by carrier gas.

The highest WHSV used in any example was only about 0.58 grams of alkyl glycolate fed per hour per gram of catalyst. Such low values of WHSV result in an undesirably low rate of alkyl glyoxylate production from a given reaction volume. The lowest degree of dilution by carrier gas used in any example was about 8 moles per mole of alkyl glycolate and the preferred range of dilution is said to be 40 - 60 moles of carrier gas per mole of glycolate. Such high dilutions result in considerable difficulty in condensing and recovering the product emerging from the reactor, as well as requiring large equipment for handling such volumes of carrier gas.

Now, according to the process of the present invention, alkyl glyoxylate is produced in high yield from alkyl glycolate under more advantageous conditions. A high WHSV of 5 or more is used, resulting in high reactor productivity. Dilution by carrier gas can be as low as about 1.2 moles $N_2$ per mole of alkyl glycolate with good results. Another advantage of the present invention is the use of readily available catalytic material which does not require further processing onto a substrate or the addition of promoters or the like.

These and other advantages of the present invention will become apparent from the following description.

## SUMMARY OF THE INVENTION

This invention provides a process for preparing alkyl glyoxylate which comprises contacting gaseous alkyl glycolate in the presence of a gaseous oxygen source with unsupported metallic silver at a temperature in the range of about $325^{\circ}$ to about $685^{\circ}$C.

The alkyl glycolate reactant has the structure $HOCH_2COOR$, wherein R is alkyl, preferably lower alkyl of 1 through 4 carbon atoms. Methyl glycolate is particularly preferred.

### DETAILED DESCRIPTION OF THE INVENTION

The catalyst used in accordance with this invention is unsupported metallic silver. In the process of this invention, the use of unsupported metallic silver offers one or more advantages over other catalysts such as supported silver dispersed on a catalyst support material, metal oxides, salts of acids derived from metal oxides, other catalysts dispersed on a catalyst support material, etc. Such advantages include high reaction selectivity, high weight hourly space velocity (WHSV) and very high thermal conductivity within the catalyst bed which allows adequate control of catalyst temperature without resorting to extreme dilution of the reaction mixture by a carrier gas and/or added inert substance.

Any number of physical forms of unsupported metallic silver are suitable, such as silver wool, chopped silver wool, silver sieves, silver tubes, or silver needles. High purity silver needles, such as the 99.999 percent pure silver needles available commercially from Engelhard Minerals & Chemicals Corp. are preferred. The preferred particle size range is 16 - 60 mesh (USSS), 1.19 mm - 0.25 mm opening, although sizes outside that range can be used. Especially preferred is a catalyst bed of two layers, with 16 - 30 mesh (1.19 mm - 0.59 mm opening) particles in the downstream layer and 30 - 60 mesh (0.59 mm - 0.25 mm opening) particles in the upstream layer. As catalyst particle size increases above the preferred range, reactivity declines and it becomes more difficult to start the reaction. As particle size declines below the preferred range, pressure drop across the catalyst bed may become excessive at the desired flow rates.

While many configurations can be used for the catalyst bed and for the reaction vessel which contains the catalyst bed, the preferred configuration comprises a shallow, horizontal, fixed catalyst bed held in place from below by a suitable grid or screen, with the reaction mixture flowing downward through the catalyst bed and screen. Among the advantages of this arrangement are a low degree of movement or disturbance of the catalyst particles by the flow of the reactant feed and avoidance of contact between the catalyst bed and the condensed reaction products when the reaction mixture is cooled and partially condensed immediately after leaving the catalyst bed.

Although it is not required, it is advantageous to place a layer of inert particles in contact with the catalyst bed on the feed side. Glass fragments in the 8 - 20 mesh (2.38 mm - 0.84 mm opening) particle size range can be used effectively for this purpose. Inclusion of such a layer tends to make the operation of the reactor more stable. The most effective thickness of such a layer can be determined by routine experimentation in view of the present description.

The metallic silver catalyst bed can be of various depths. Good results have been obtained with bed depths in the range of 3 - 40 mm, and a bed depth of 10 - 30 mm is preferred. As the bed depth declines below the preferred range, the operation becomes less stable, and as bed depth increases above the range, excessive reaction can occur with reduced reaction selectivity and the pressure drop across the catalyst bed can become excessive.

Control of the temperature of the catalyst bed is very important in order to obtain good reaction selectivity. While catalyst bed temperatures as low as $325^{o}$C and as high as $685^{o}$C can be employed, temperatures in the range of $400^{o}$ to $610^{o}$C are preferred. It is also preferred that the catalyst temperature be constant and as uniform as possible along directions perpendicular to the flow of the reactants. As catalyst temperature declines below the preferred range, it becomes more difficult to maintain stable reactor operation. As catalyst temperature rises above the range side reactions become excessive. Among the effective means of controlling catalyst temperature are adjustment of the feed temperature, dilution of the feed with relatively inert materials and cooling applied to the downstream surface of the catalyst bed. Cooling applied to the outer edges of the bed is less desirable because of its tendency to create temperature gradients perpendicular to the direction of the flow of the reactants. Since the reaction is highly exothermic, as the degree of conversion of the alkyl glycolate is increased, the catalyst temperature tends to increase unless other adjustments are simultaneously made to prevent a rise in catalyst temperature.

The oxygen source for this process can be oxygen or an oxygen containing gas, for example, air. The mole ratio of oxygen to alkyl glycolate can vary widely, for example, from about 0.05 to about 1.00 or more. It is preferred to use air to provide an oxygen to glycolate mole ratio in the range of about 0.3 to about 0.7. If the oxygen source is mixed with previously vaporized alkyl glycolate, it is preferred to preheat it to a temperature above the condensation point of the alkyl glycolate in order to avoid formation of mist.

It is important that the mixture fed to the catalyst bed is uniformly mixed and completely in the gaseous state. Various means of accomplishing this will be apparent to those skilled in the art. For example, the alkyl glycolate along with any other liquid components can be vaporized in an upflow long-tube vertical evaporator, freed of mists by means of a suitable fiber bed, and then mixed uniformly with preheated air at a temperature high enough to avoid mist formation. Failure to eliminate the mist or entrained liquid can adversely affect both reaction selectivity and catalyst life.

The alkyl glycolate feed material for the process of this invention can be prepared by the esterification of glycolic acid with an aliphatic alcohol. The aliphatic alcohol can be straight or branched chain and preferably contains from 1 to about 4 carbon atoms. Methanol is particularly preferred. The alkyl glycolate can be separated from alcohol and water present in the esterification reaction mixture prior to use in the process of this invention. Alternatively, the alcohol and water can remain with the alkyl glycolate fed in the present process. When methanol is present, it is possible to adjust operating conditions in such a way that only a small fraction of the methanol is oxidized. Any harmful levels of catalyst poisoning impurities must be removed from the feed mixture prior to its contact with the silver catalyst.

The weight hourly space velocity (WHSV) or weight of alkyl glycolate fed per hour per unit weight of catalyst affects the selectivity of the reaction. Relatively high values of WHSV are desirable in this process both because of improved reaction selectivity and because of increased product output from a given amount of catalyst. Although WHSV can vary from 5 to 905 or even more widely, the range of 10 to 850 is preferred and the range of 15 to 500 is especially preferred. As WHSV increases above the range, it becomes more difficult to avoid excessively high catalyst temperatures, excessively high pressure drop across the catalyst bed or instability of the reaction. As WHSV declines below the range, side reactions tend to become excessive.

The degree of conversion of alkyl glycolate to alkyl glyoxylate per pass in the process of this invention can be varied over a wide range. Conversions in the range of 20 to 70 percent are preferred. At conversions below the preferred range, the output of alkyl glyoxylate from equipment of a given size is unnecessarily and objectionably low, and energy consumption for feed vaporization and alkyl glyoxylate separation is excessive. As conversion increases above the preferred range, it becomes increasingly more difficult to avoid excessive catalyst temperature and objectionably low reaction selectivity. The degree of glycolate conversion can be controlled by means of adjustments in the amount of oxygen fed to the reactor, the catalyst temperature and the WHSV. It is possible to achieve a high degree of reaction selectivity, i.e. at least 80 percent selectivity to alkyl glyoxylate based upon alkyl glycolate converted over the entire preferred range of glycolate conversion.

The degree of conversion of the oxygen fed to this process can also be varied widely. Good selectivity in the conversion of alkyl glycolate to alkyl glyoxylate has been observed at oxygen conversion levels varying from about 60 percent to about 98 percent. Selectivity tends to improve as oxygen conversion declines within this range and good selectivity is also expected at still lower oxygen conversions. However, very low oxygen conversions are less desirable because the volume of oxygen containing gas to be handled is excessive and because explosive mixtures are more likely to be encountered. Also, as oxygen conversion declines, it becomes more difficult to maintain close control over conversions of either oxygen or glycolate. In view of such considerations, the preferred range for oxygen conversion is approximately 60 to 95 percent. Even more preferred is the range of 70 to 90 percent. Control of oxygen conversion at a desired level requires the proper adjustment of a number of interacting parameters such as the $O_2$/glycolate mole ratio, level of diluents, catalyst cooling mechanism and conditions, feed temperature, WHSV, and catalyst history. The required adjustment can be determined by routine experimentation in view of the present description.

Good results have been obtained over a wide range of pressure drop across the reaction system. Pressure drop across the feed vaporization system and reactor combined can be as high as 80 psi (551 kPa) or as low as 3 psi (20.7 kPa) without large adverse effects. However, increases in pressure drop which are not associated with one or more beneficial changes in other parameters, such as an increase in WHSV to a more favorable value, can result in lower reaction selectivity.

During start-up of the reactor, it is important to prevent both excessively high transient catalyst temperature and oxygen deficient conditions which can cause coke deposition upon the catalyst. Various procedures for avoiding such problems during start-up will be apparent to those skilled in the art.

While it is possible to use additional diluents or carrier gases beyond the nitrogen from the air fed and any alcohol and water remaining from the preparation of alkyl glycolate by esterification, it is preferred to avoid the use of such additional diluents and good results can be obtained without them in the present process.

The reactor, catalyst bed screen, feed vaporization system and product collection system can be fabricated of various materials resistant to corrosive attack by the process streams at the temperatures employed and free of any tendency to cause rapid poisoning of the silver catalyst. Satisfactory results have been obtained with equipment made of stainless steel, particularly 316 stainless steel and Carpenter 20.

Rapid cooling of the product stream as it emerges from the catalyst bed is important in order to minimize side reactions. While this can be accomplished in various ways, in the preferred reactor configuration, a cooling surface is located immediately below the catalyst bed screen. This cooling surface can be either slightly separated from or in contact with the catalyst support screen, depending upon how much heat removal from the catalyst bed itself is desired. As the product stream is rapidly cooled to a temperature below the condensation point of the products in the stream, a portion of the condensate may form an aerosol which is difficult to recover. This problem is readily solved by passing the stream through a suitable fiber bed to coalesce the aerosol. Since a considerable amount of heat is generated by the reaction, it is desirable to remove it in such a way that some of it can be utilized elsewhere in the process, such as for vaporization of the alkyl glycolate feed. Suitable arrangements to accomplish this and to complete the cooling, condensation and collection of the condensable reaction products will be apparent to those skilled in the art in view of the present description. The alkyl glyoxylate product can be separated by distillation from the condensate.

This invention is further illustrated by, but not limited to, the following examples wherein all percentages and parts are by weight unless otherwise indicated.

## EXAMPLE I

A liquid mixture containing 60.4% methyl glycolate, 18.1% water and 21.5% methanol was continuously pumped into an electrically heated vaporizer by means of a recriprocating piston pump rated for pumping against back pressures up to 100 PSIG (791.5 kPa). The mixture was completely vaporized within the vaporizer and the resulting gaseous mixture was sent through a glass fiber mist eliminator to a mixing zone where it was mixed with air preheated to about $170^\circ - 180^\circ C$, in amounts sufficient to produce a mole ratio of $O_2$ to methyl glycolate of 0.50. The temperature of the resulting mixture was adjusted to $220^\circ C$ and the mixture was fed to the top of a vertical reactor constructed of 316 stainless steel tubing, 0.25 inch (6.35 mm) OD.

Within the reactor, the reaction mixture passed downward through a layer of 10 - 12 mesh (2.0 - 1.68 mm opening) glass fragments, then through a layer consisting of 1.50 grams of 30 - 60 mesh (0.59 - 0.25 mm opening) silver needles (Engelhard Mineral & Chemical Corp., 99.999% purity) held in place from below with a 316 stainless steel screen at the bottom of the reaction chamber. Total depth of the glass fragments plus catalyst was about 60 mm. The reactor was insulated and electrically heated to prevent excessive heat loss through its wall. The temperature of the silver catalyst was maintained at about 600°C and the depth of the silver catalyst bed was about 23 mm. The methyl glycolate feed rate was adjusted to produce a weight hourly space velocity (WSHV) of 348 grams of methyl glycolate per gram of catalyst per hour. The pressure drop measured across the vaporizer and reactor combined was 80 psi (551 kPa).

The product stream emerging from the bottom of the reaction chamber was passed over a 316 stainless steel cold finger with its top surface 2 mm from the catalyst bed screen. The cold finger was water cooled and had an outer diameter of 0.5 inch (12.7 mm). The product stream was then sent through a further series of coolers and a mist eliminator in order to complete the condensation, cooling and collection of the condensable components.

The products were weighed and analyzed by gas chromatography. It was found that 50.7% of methyl glycolate fed was consumed and 85.3% of the consumed methyl glycolate was converted to recovered methyl glyoxylate. The amount of methanol recovered was equivalent to 90.5% of the sum of the methanol fed and an estimated 1 mole of methanol formed within the reactor per mole of alkyl glycolate converted to by-products.

### EXAMPLES II - VIII

Results from a series of seven consecutive runs are tabulated in Table I. The operating procedure of Example 1 was used except for the variations noted in the table. In addition, in these runs the oxygen conversion was monitored by means of an in line oxygen analyzer with the results indicated in the table. These runs, listed in order of decreasing selectivity, illustrate the principle that within the preferred ranges of the operating conditions, average selectivity in converting methyl glycolate to methyl glyoxylate is improved by operating at a lower level of oxygen conversion. In the runs the pressure drop ranged from about 34 - 56 psi (234.4 - 386.1 kPa) across the vaporizer and reactor combined.

## TABLE I

| Example Number | II | III | IV | V | VI | VII | VIII |
|---|---|---|---|---|---|---|---|
| Catalyst Weight (g) | 0.772 | 0.808 | 1.00 | 0.808 | 0.772 | 0.772 | 0.772 |
| Catalyst Depth (mm) | 12 | 12 | 16 | 12 | 12 | 12 | 12 |
| WHSV | 831 | 736 | 636 | 464 | 905 | 875 | 493 |
| $O_2$/glycolate (molar) | 0.37 | 0.42 | 0.30 | 0.41 | 0.34 | 0.35 | 0.47 |
| Feed Temperature (°C) | 245 | 270 | 240 | 260 | 240 | 240 | 231 |
| Catalyst Temperature (°C) | 470 | 475 | 550 | 520 | 555 | 565 | 550 |
| Pressure Drop (psi) | 44 | 56 | 52 | 34 | 56 | 48 | 36 |
| % Glycolate Conversion | 27.5 | 21.4 | 38.6 | 32.1 | 41.8 | 46.7 | 56.8 |
| % Oxygen Conversion | 62 | 70 | 98 | 71 | 96 | 97 | 95 |
| % Selectivity | 88.0 | 87.0 | 80.5 | 79.7 | 78.6 | 77.7 | 67.8 |
| % Methanol Recovery | 100 | 100 | 97.7 | 100 | 91.0 | 86.8 | 82.6 |

## EXAMPLE IX

Methyl glycolate was converted to methyl glyoxylate under conditions comparable to those in the preceding examples except that the $O_2$/glycolate mole ratio was increased to 0.62, the WHSV employed was at 253, and the catalyst bed consisted of a lower layer of 16 - 30 mesh (1.19 - 0.59 mm opening) silver needles weighing 1.00 gram and an upper layer of 30 - 60 mesh (0.59 - 0.25 mm opening) silver needles weighing 0.50 gram. Total catalyst bed depth was 22 mm. In this run, 69.4% of the methyl glycolate was consumed, and 89.3% of the consumed methyl glycolate was converted to recovered methyl glyoxylate. This illustrates the good results which can be obtained in this process using a layered catalyst bed. It also illustrates that good selectivity can be maintained at a glycolate conversion level of nearly 70%.

## EXAMPLE X

Methyl glycolate was converted to methyl glyoxylate by a method comparable to that of the preceding examples except that the % glycolate conversion was increased to 94.3% and the catalyst temperature was increased to 685°C. In this run the selectivity in converting glycolate to glyoxylate was only 30.6%. This illustrated the adverse effects which can result from operation at catalyst temperatures and glycolate conversion levels well above the ranges preferred in this process.

## EXAMPLE XI

Methyl glycolate was converted to methyl glyoxylate by a method comparable to that of Examples I-IX except that the feed contained less than 1.5% water and methanol, the WHSV was about 32, and the catalyst temperature was 425°C. Consumption of glycolate averaged 31.6% and selectivity to glyoxylate averaged 83.6%. This illustrates that good selectivity can be obtained in this process within the lower portion of the preferred range of WHSV by using a relatively low catalyst temperature within the preferred range for catalyst temperature.

### EXAMPLE XII

Methyl glycolate was converted to methyl glyoxylate by a method comparable to that of Example XI except that the WHSV was 197 and the catalyst temperature was $565^{\circ}C$. The pressure drop was only 15 psi (103.4 kPa), the glycolate conversion was 62.8% and the selectivity to glyoxylate was 82.5%. This illustrates that good selectivity can be obtained under conditions resulting in a relatively low pressure drop.

### EXAMPLE XIII

Methyl glycolate was converted to methyl glyoxylate by a method comparable to that of Examples I - IX. The $O_2$/glycolate molar ratio was 0.31, the glycolate conversion was 36.3% and the selectivity to glyoxylate was 84.0%. This illustrates that high selectivity can be obtained with the $O_2$/glycolate ratio near the low end of its preferred range.

The following examples further illustrate the advantage of this invention employing a larger reactor which is believed to be more representative of conducting the process on a commercial scale at reduced pressure drops.

### EXAMPLE IV

Methyl glycolate was converted to methyl glyoxylate by a method comparable to that of Example XI except that a larger reaction system was employed, WHSV was about 18 and the catalyst temperature was $530^{\circ}C$. The outer diameter of the reaction chamber employed was one inch (2.54 cm) instead of 0.25 inch (6.35 mm) and weight of catalyst employed was 24 grams to provide a bed depth of about 22 mm. The $O_2$/glycolate molar ratio was 0.40 and the pressure drop across the catalyst bed alone 2 psi (13.8 kPa). The pressure drop across the vaporizer in reactor combined was 6.5 psi (44.8 kPa). Oxygen conversion was 92%, glycolate conversion was 69.6% and the selectivity to glyoxylate was 82.3%. This illustrates that good selectivity can be achieved in this process in the lower portion of the preferred range of WHSV and under conditions resulting in a relatively low pressure drop.

## EXAMPLE XV

Methyl glycolate was converted to methyl glyoxylate by the procedure of Example XIV except that the WHSV was reduced to 8 and catalyst temperature was 460°C. The pressure drop across the catalyst bed was 1.5 psi (10.3 kPa), and the pressure drop across the vaporizer and reactor combined was 4.0 psi (27.6 kPa). The $O_2$/glycolate molar ratio was 0.38 and the oxygen conversion was 75%. Glycolate conversion was 43.7% and the selectivity to glyoxylate was 78.8%. This illustrates some decline in selectivity when the WHSV is below the preferred range.

Although the invention has been described in terms of specified embodiments which are set forth in considerable detail, it should be understood that this is by way of illustration only and that the invention is not necessarily limited thereto since alternative embodiments and operating techniques will become apparent to those skilled in the art in view of the disclosure. Accordingly, modifications are contemplated which can be made without departing from the spirit of the described invention.

WHAT IS CLAIMED IS:

1. A process for preparing alkyl glyoxylate which comprises contacting gaseous alkyl glycolate in the presence of a gaseous oxygen source with unsupported metallic silver at a temperature in the range of about $325^{o}$ to about $685^{o}C$.

2. The process of Claim 1 wherein the metallic silver is in the form of silver wool, chopped silver wool, silver sieves, silver tubes or silver needles.

3. The process of Claim 1 wherein the alkyl glycolate is lower alkyl glycolate.

4. The process of Claim 1 wherein the temperature is in the range of from $400^{o}C$ to $610^{o}C$.

5. The process of Claim 1 wherein the oxygen source is air and the alkyl glycolate is methyl glycolate.

6. The process of Claim 5 wherein the air present is sufficient to provide about 0.3 to about 0.7 mole of oxygen per mole of methyl glycolate.

7. A process for preparing lower alkyl glyoxylate which comprises contacting a gaseous mixture comprising lower alkyl glycolate and an oxygen source with metallic silver by passing the gaseous mixture downwardly through a fixed bed of silver needles having a bed depth of about 3 to about 40 mm and a bed temperature in the range of about $325^{o}$ to about $685^{o}C$.

8. The process of Claim 7 wherein the oxygen source is air in an amount sufficient to provide about 0.3 to about 0.7 mole of oxygen per mole of alkyl glycolate and the lower alkyl glycolate is methyl glycolate.

9. The process of Claim 8 wherein the silver needles have a particle size 16 - 60 mesh USSS, 1.19 mm - 0.25 mm opening.

10. The process of Claim 8 wherein the bed depth is about 10 mm to about 30 mm.

11.  The process of Claim 8 wherein the bed temperature is in the range of 400$^{o}$ to 610$^{o}$C.

12.  The process of Claim 8 wherein the amount of methyl glycolate converted is in the range of 20 to 70 percent by weight.

13.  A process for preparing methyl glyoxylate which comprises:  contacting a gaseous mixture comprising methyl glycolate and an oxygen source with metallic silver by passing the gaseous mixture downwardly through a fixed catalyst bed of silver needles having a bed depth of about 3 to about 40 mm and a bed temperature in the range of about 325$^{o}$ to about 685$^{o}$C to form a product stream; cooling the product stream to form a condensate and recovering methyl glyoxylate from the condensate.

14.  The process of Claim 13 wherein the gaseous mixture is passed at a weight hourly space velocity in the range of about 5 to about 905 and the oxygen source is air in an amount sufficient to provide about 0.3 to about 0.7 mole of oxygen per mole of methyl glycolate.

15.  The process of Claim 14 wherein the weight hourly space velocity is in the range of 10 to 850.

16.  The process of Claim 14 wherein the bed depth is in the range of 10 mm to 30 mm and the bed temperature is in the range of 400$^{o}$ to 610$^{o}$C.

17.  The process of Claim 14 wherein the amount of methyl glycolate converted is in the range of 20 to 70 percent by weight and the amount of oxygen converted is in the range of 60 to 95 percent by weight.

18.  The process of Claim 17 wherein a layer of inert particles is in contact with the catalyst bed on the feed side.

19. The process of Claim 18 wherein the gaseous mixture further comprises methanol and water.

20. The process of Claim 18 wherein a mist eliminator is employed during the cooling of the product stream to form the condensate.